(19) Europäisches Patentamt
European Patent Office
Office européen des brevets

(11) Veröffentlichungsnummer: **0 591 699 A1**

(12) # EUROPÄISCHE PATENTANMELDUNG

(21) Anmeldenummer: **93114541.1**

(22) Anmeldetag: **10.09.93**

(51) Int. Cl.⁵: **C07D 213/82**, C07D 277/56, A01N 43/40, A01N 43/78

(30) Priorität: **21.09.92 DE 4231518**

(43) Veröffentlichungstag der Anmeldung: **13.04.94 Patentblatt 94/15**

(84) Benannte Vertragsstaaten: **AT BE CH DE DK ES FR GB GR IE IT LI NL PT SE**

(71) Anmelder: **BASF Aktiengesellschaft**
**Carl-Bosch-Strasse 38**
**D-67063 Ludwigshafen(DE)**

(72) Erfinder: **Eicken, Karl, Dr.**
**Am Huettenwingert 12**
**D-6706 Wachenheim(DE)**
Erfinder: **Ammermann, Eberhard, Dr.**
**Von-Gagern-Strasse 2**
**D-6148 Heppenheim(DE)**
Erfinder: **Lorenz, Gisela, Dr.**
**Erlenweg 13**
**D-6730 Neustadt(DE)**

(54) **N-Hydroxy-N-phenylcarbonsäureamide, Verfahren zu ihrer Hestellung und sie enthaltende Mittel zur Bekämpfung von Schadpilzen.**

(57) 1. N-Hydroxy-N-phenylcarbonsäureamide der Formel I

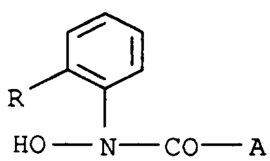

I

in der die Substituenten die folgende Bedeutung haben:

R ggf. subst. Alkyl, Alkoxy, Alkenyl, Alkenyloxy, Alkinyl, Alkinyloxy, Cycloalkyl, Cycloalkenyl, Cycloalkyloxy, Cycloalkenyloxy oder Phenyl;

A einer der Reste A1 bis A7

EP 0 591 699 A1

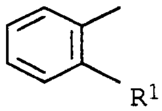

A1

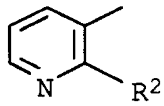

A2

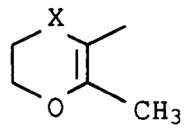

A3

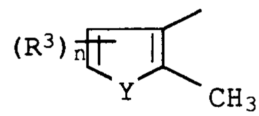

A4

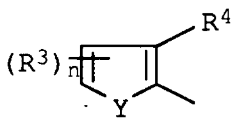

A5

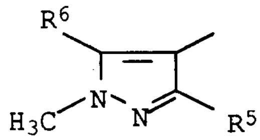

A6

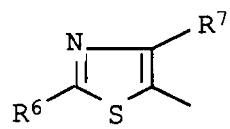

A7

mit

| | |
|---|---|
| X | $-CH_2-$, -S-, -SO- oder $-SO_2-$; |
| Y | -O- oder -S-; |
| $R^1$, $R^2$, $R^4$, $R_5$ und $R^7$ | Halogen, Alkyl oder Halogenalkyl; |
| $R^3$ und $R^6$ | Wasserstoff, Halogen oder Alkyl; |
| n | 1 oder 2; |

Verfahren zu ihrer Herstellung, sowie sie enthaltende Mittel und deren Verwendung zur Bekämpfung von Schadpilzen.

Die vorliegende Erfindung betrifft N-Hydroxy-N-phenylcarbonsäureamide der Formel I

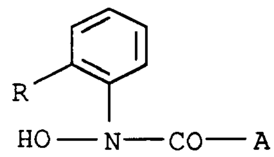

I

in der die Substituenten die folgende Bedeutung haben:

R — $C_2$-$C_{12}$-Alkyl, $C_2$-$C_{12}$-Alkoxy, $C_3$-$C_{12}$-Alkenyl, $C_3$-$C_{12}$-Alkenyloxy, $C_3$-$C_6$-Alkinyl, $C_3$-$C_6$-Alkinyloxy, wobei diese Gruppen partiell oder vollständig halogeniert sein können; $C_3$-$C_7$-Cycloalkyl, $C_4$-$C_7$-Cycloalkenyl, $C_3$-$C_7$-Cycloalkyloxy oder $C_4$-$C_7$-Cycloalkenyloxy, wobei diese Ringe ein bis 3 $C_1$-$C_4$-Alkylgruppen tragen können; Phenyl, welches ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

A — ein cyclischer Rest aus der Gruppe der Formeln A1 bis A7:

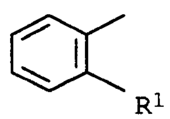

A1

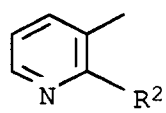

A2

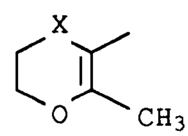

A3

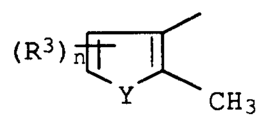

A4

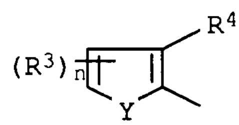

A5

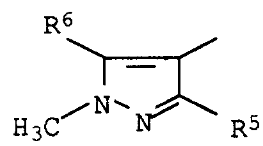

A6

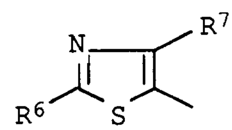

A7

in denen die Substituenten die folgende Bedeutung haben:

X — $-CH_2-$, -S-, -SO- oder $-SO_2-$;

Y — -O- oder -S-;

$R^1$, $R^2$, $R^4$, $R_5$ und $R^7$ — Halogen, $C_1$-$C_4$-Alkyl oder $C_1$-$C_4$-Halogenalkyl;

$R^3$ und $R^6$ — Wasserstoff, Halogen oder $C_1$-$C_4$-Alkyl;

n — 1 oder 2, wobei die Reste $R^3$ verschieden sein können, wenn der Wert von n 2 beträgt.

Außerdem betrifft die Erfindung Verfahren zur Herstellung dieser Verbindungen, sie enthaltende Mittel und Verfahren zu deren Verwendung zur Bekämpfung von Schadpilzen, insbesondere von Botrytis.

Aus der Literatur ist 2-Chlornicotinsäure-2-chloranilid als fungizider Wirkstoff bekannt (DE-A 24 17 216).

Aufgabe der vorliegenden Erfindung waren neue fungizid wirksame Verbindungen mit verbessertem Wirkungsspektrum.

Demgemäß wurden die eingangs definierten Verbindungen I gefunden.

Außerdem wurden Verfahren zur Herstellung dieser Verbindungen, sie enthaltende Mittel und Verfahren zu deren Verwendung zur Bekämpfung von Schadpilzen gefunden.

Ma erhält die Verbindungen I im allgemeinen dadurch, daß man ein Carbonsäurehalogenid der Formel II in an sich bekannter Weise (z.B. J. March, Advanced Organic Chemistry, 2nd Ed., 382 f, McGraw-Hill, 1977) in Gegenwart einer Base mit einem N-Hydroxyanilin der Formel III umsetzt.

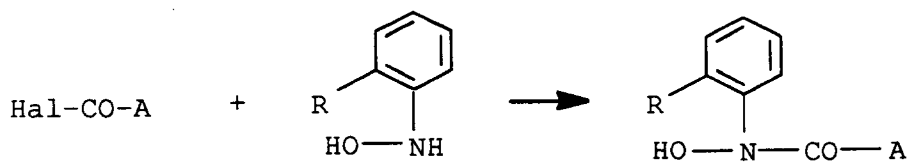

II III I

Der Rest Hal in der Formel II steht für ein Halogenatom wie Chlor, Brom und Jod, insbesondere Chlor oder Brom.

Diese Umsetzung erfolgt üblicherweise bei Temperaturen von -20 °C bis 100 °C, vorzugsweise -10 °C bis 50 °C.

Geeignete Lösungsmittel sind:
Aliphatische Kohlenwasserstoffe wie Pentan, Hexan, Cyclohexan und Petrolether, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol sowie Dimethylsulfoxid und Dimethylformamid, besonders bevorzugt Toluol, Xylol und Tetrahydrofuran.

Es können auch Gemische der genannten Lösungsmittel verwendet werden.

Als Basen kommen allgemein anorganische Verbindungen wie Alkalimetall-und Erdalkalimetallhydroxide wie Lithiumhydroxid, Natriumhydroxid, Kaliumhydroxid und Calziumhydroxid, Alkalimetall- und Erdalkalimetalloxide wie Lithiumoxid, Natriumoxid, Calziumoxid und Magnesiumoxid, Alkalimetall- und Erdalkalimetallhydride wie Lithiumhydrid, Natriumhydrid, Kaliumhydrid und Calziumhydrid, Alkalimetallamide wie Lithiumamid, Natriumamid und Kaliumamid, Alkalimetall- und Erdalkalimetallcarbonate wie Lithiumcarbonat und Calziumcarbonat sowie Alkalimetallhydrogencarbonate wie Natriumhydrogencarbonat, und metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium-tert.-Butanolat und Dimethoxymagnesium außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydrogencarbonat, Natriumcarbonat, Triethylamin und Pyridin.

Die Basen werden im allgemeinen in äquimolarem Mengen bezogen auf die Verbindung II eingesetzt. Sie können aber auch in einem Überschuß von 5 mol-% bis 30 mol-%, vorzugsweise 5 mol-% bis 10 mol-%, oder - im Falle der Verwendung von tertiären Aminen - gegebenenfalls als Lösungsmittel verwendet werden.

Die Edukte werden im allgemeinen in äquimolaren Mengen miteinander umgesetzt. Es kann für die Ausbeute vorteilhaft sein, II in einem Überschuß von 1 mol-% bis 20 mol-%, vorzugsweise 1 mol-% bis 10 mol-%, bezogen auf III einzusetzen.

Die für die Herstellung der Verbindungen I benötigten Ausgangsstoffe der Formel II und III sind in der Literatur bekannt (Houben Weyl, Methoden der org. Chemie, Bd. 10/1, S. 1138-1148) oder können gemäß der zitierten Literatur hergestellt werden.

Im Hinblick auf ihre Verwendung in fungiziden Mitteln kommen Verbindungen der Formel I in Betracht, in der die Substituenten die folgende Bedeutung haben:

R $C_2$-$C_{12}$-Alkyl wie Ethyl und geradkettiges oder verzweigtes Propyl, Butyl, Pentyl, Hexyl, Heptyl, Octyl, Nonyl, Decyl, Undecyl und Dodecyl, besonders geradkettiges oder verzweigtes $C_3$-$C_{10}$-Alkyl wie Propyl, 1-Methylethyl, Butyl, 1-Methylpropyl, 2-Methylpropyl, 1,1-Dimethylethyl, n-Pentyl, 1-Methylbutyl, 2-Methylbutyl, 3-methylbutyl, 1,2-Dimethylpropyl, 1,1-Dimethylpropyl, 2,2-Dimethylpropyl, 1-Ethylpropyl, n-Hexyl, 1-Methpentyl, 2-Methylpentyl, 3-Methylpentyl, 4-Methylpentyl, 1,2-Dimethylbutyl, 1,3-Dimethylbutuyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-3-methylpropyl, n-Heptyl, 1-Methylhexyl, 1-Ethylpentyl, 2-Ethylpentyl, 1-Propylbutyl, Octyl, 1-Methylheptyl, 2-Methylheptyl, 1-Ethylhexyl, 2-Ethylhexyl, 1-Propylpentyl, 2-Propylpentyl, Nonyl, 1-Methyloctyl, 2-Methyloctyl, 1-Ethylheptyl, 2-Ethylheptyl, 1-Propylhexyl, 2-Propylhexyl, Decyl, 1-Methylnonyl, 2-Methylnonyl, 1-Ethyloctyl, 2-Ethyloctyl, 1-Propylheptyl und 2-Propylheptyl, insbesondere Propyl, 1-Methylethyl, Butyl, 1-Methylbutyl, 2-Methylbutyl, 1,1-Dimethylethyl, Pentyl, 1-Methylbutyl, Hexyl, Heptyl und 1-Methylheptyl, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

$C_2$-$C_{12}$-Alkoxy wie Ethoxy und geradkettiges oder verzweigtes Propyloxy, Butyloxy, Pentyloxy, Hexyloxy, Heptyloxy, Octyloxy, Nonyloxy, Decyloxy, Undecyloxy und Dodecyloxy, besonders geradkettiges oder verzweigtes $C_2$-$C_{10}$-Alkoxy wie Ethoxy, Propoxy, 1-Methylethoxy, Butoxy, 1-Methylpropoxy, 2-Methylpropoxy, 1,1-Dimethylethoxy, n-Pentyloxy, 1-Methylbutoxy, 2-Methylbutoxy, 3-Methylbutoxy, 1,2-Dimethylpropoxy, 1-Ethylpropoxy, n-Hexyloxy, 1-Methylpentyloxy, 2-Methylpentyoxy, 3-Methylpentyloxy, 4-Methylpentyloxy, 1,2-Dimethylbutoxy, 1,3-Dimethylbutoxy, 2,3-Dimethylbutoxy, 1,2-Dimethylbutoxy, 2,2-Dimethylbutoxy, 3,3-Dimethylbutoxy, 1,1,2-Trimethylpropoxy, 1,2,2-Trimethylpropoxy, 1-Ethylbutoxy, 2-Ethylbutoxy, 1-Ethyl-2-methylpropoxy, n-Heptyloxy, 1-Methylhexyloxy, 2-Methylhexyloxy, 3-Methylhexyloxy, 4-Methylhexyloxy, 5-Methylhexyloxy, 1-Ethylpentyloxy, 2-Ethylpentyloxy, 1-Propylbutoxy, Octyloxy, 1-Methylheptyloxy, 2-Methylheptyloxy, 1-Ethylhexyloxy, 2-Ethylhexyloxy, 1-Propylpentyloxy, 2-Propylpentyloxy, Nonyloxy, 1-Methyloctyloxy, 2-Methyloctyloxy, 1-Ethylheptyloxy, 2-Ethylheptyloxy, 1-Propylhexyloxy, 2-Propylhexyloxy, Decyloxy, 1-Methylnonyloxy, 2-Methylnonyloxy, 1-Ethyloctyloxy, 2-Ethyloctyloxy, 1-Propylheptyloxy und 2-Propylheptyloxy, insbesondere Ethoxy, Propyloxy, 1-Methylethoxy, Butyloxy, 1-Methylpropyloxy, 2-Methylpropyloxy, 1,1-Dimethylethoxy, Pentyloxy, Hexyloxy und 2-Ethylhexyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise Halogenalkoxy wie Chlormethyloxy, Dichlormethyloxy, Trichlormethyloxy, Fluormethyloxy, Difluormethyloxy, Trifluormethyloxy, Chlorfluormethyloxy, Dichlorfluormethyloxy, Chlordifluormethyloxy, 1-Fluorethyloxy, 2-Fluorethyloxy, 2,2-Difluorethyloxy, 2,2,2-Trifluorethyloxy, 2-Chlor-2-fluorethyloxy, 2-Chlor-2,2-difluorethyloxy, 2,2-Dichlor-2-fluorethyloxy, 2,2,2-Trichlorethyloxy und Pentafluorethyloxy;

$C_3$-$C_{12}$-Alkenyl wie geradkettiges oder verzweigtes Propenyl, Butenyl, Pentenyl, Hexenyl, Heptenyl, Octenyl, Nonenyl, Decenyl, Undecenyl und Dodecenyl, besonders gradkettiges oder verzweigtes $C_3$-$C_{10}$-Alkenyl wie 2-Propenyl, 2-Butenyl, 3-Butenyl, 1-Methyl-2-propenyl, 2-Methyl-2-propenyl, 2-Pentenyl, 3-Pentenyl, 4-Pentenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-2-propenyl, 1-Ethyl-2-propenyl, 2-Hexenyl, 3-Hexenyl, 4-Hexenyl, 5-Hexenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-2-butenyl, 1,2-Dimethyl-3-butenyl, 1,3-Dimethyl-2-butenyl, 1,3-Dimethyl-3-butenyl, 2,2-Dimethyl-3-butenyl, 2,3-Dimethyl-2-butenyl, 2,3-Dimethyl-3-butenyl, 1-Ethyl-2-butenyl, 1-Ethyl-3-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-2-propenyl, 1-Methyl-2-pentenyl, 2-Methyl-2-pentenyl, 1-Methyl-3-pentenyl, 2-Methyl-3-pentenyl, 1-Methyl-2-hexenyl, 2-Methyl-2-hexenyl, 1-Methyl-3-hexenyl, 2-Methyl-3-hexenyl, 1-Ethyl-2-pentenyl, 2-Ethyl-2-pentenyl, 1-Ethyl-3-pentenyl, 2-Ethyl-3-pentenyl, 1-Methyl-2-heptenyl, 2-Methyl-2-heptenyl, 1-Methyl-3-heptenyl, 2-Methyl-3-heptenyl, 1-Ethyl-2-hexenyl, 2-Ethyl-2-hexenyl, 1-Ethyl-3-hexenyl, 2-Ethyl-3-hexenyl, 1-Methyl-2-octenyl, 2-Methyl-2-octenyl, 1-Methyl-3-octenyl, 2-Methyl-3-octenyl, 1-Ethyl-2-heptenyl, 2-Ethyl-2-heptenyl, 1-Ethyl-3-heptenyl, 2-Ethyl-3-heptenyl, 1-Ethyl-2-octenyl, 2-Ethyl-2-octenyl, 1-Ethyl-3-octenyl und 2-Ethyl-3-octenyl, insbesondere 1-Propenyl, 2-Propenyl, 1-Methylethenyl, 1-Methyl-2-propenyl, 2-Methyl-2-propenyl, 1-Ethyl-2-propenyl, 1-Methyl-2-butenyl, 1-Ethyl-2-butenyl, 1-(1-Methylethyl)-2-butenyl, 1-Butyl-2-butenyl, 1-Methyl-2-pentenyl und 1,4-Dimethyl-2-pentenyl; wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, insbesondere 3-Chlor-2-propenyl und 2,3-Dichlor-2-propenyl;

$C_3$-$C_{12}$-Alkenyloxy wie geradkettiges oder verzweigtes Propenyloxy, Butenyloxy, Pentenyloxy, Hexenyloxy, Heptenyloxy, Octenyloxy, Nonenyloxy, Decenyloxy, Undecenyloxy und Dodecenyloxy, besonders gradkettiges oder verzweigtes $C_3$-$C_{10}$-Alkenyloxy wie 2-Propenyloxy, 2-Butenyloxy, 3-Butenyloxy, 1-Methyl-2-propenyloxy, 2-Methyl-2-propenyloxy, 2-Pentenyloxy, 3-Pentenyloxy, 4-Pentenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-2-propenyloxy, 1-Ethyl-2-propenyloxy, 2-Hexenyloxy, 3-Hexenyloxy, 4-Hexenyloxy, 5-Hexenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1,1-Dimethyl-2-butenyloxy, 1,1-Dimethyl-3-butenyloxy, 1,2-Dimethyl-2-butenyloxy, 1,2-Dimethyl-3-butenyloxy, 1,3-Dimethyl-2-butenyloxy, 1,3-Dimethyl-3-butenyloxy, 2,2-Dimethyl-3-butenyloxy, 2,3-Dimethyl-2-butenyloxy, 2,3-Dimethyl-3-butenyloxy, 1-Ethyl-2-butenyloxy, 1-Ethyl-3-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-2-propenyloxy, 1-Methyl-2-pentenyloxy, 2-Methyl-2-pentenyloxy, 1-Methyl-3-pentenyloxy, 2-Methyl-3-pentenyloxy, 1-Methyl-2-hexenyloxy, 2-Methyl-2-hexenyloxy, 1-Methyl-3-hexenyloxy, 2-Methyl-3-hexenyloxy, 1-Ethyl-2-pentenyloxy, 2-Ethyl-2-pentenyloxy, 1-Ethyl-3-pentenyloxy, 2-Ethyl-3-pentenyloxy, 1-Methyl-2-heptenyloxy, 2-Methyl-2-heptenyloxy, 1-Methyl-3-heptenyloxy, 2-Methyl-3-heptenyloxy, 1-Ethyl-2-hexenyloxy, 2-Ethyl-2-hexenyloxy, 1-Ethyl-3-hexenyloxy, 2-Ethyl-3-hexenyloxy, 1-Methyl-2-octenyloxy, 2-Methyl-2-octenyloxy, 1-Methyl-3-octenyloxy, 2-Methyl-3-octenyloxy, 1-Ethyl-2-heptenyloxy, 2-Ethyl-2-heptenyloxy, 1-Ethyl-3-heptenyloxy, 2-Ethyl-3-heptenyloxy, 1-Ethyl-2-octenyloxy, 2-Ethyl-2-octenyloxy, 1-Ethyl-3-octenyloxy und 2-Ethyl-3-octenyloxy, insbesondere 2-Propenyloxy, 1-Methyl-2-propenyloxy, 2-Methyl-2-propenyloxy, 2-Pentenyloxy, 3-Pentenyloxy, 1-Methyl-2-butenyloxy und 1-Methyl-2-pentenyloxy, wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, insbesondere 3-Chlor-2-propenyloxy, 2,3-Dichlor-2-propenyloxy und 2,3,3-Trichlor-2-propenyloxy;

$C_3$-$C_6$-Alkinyl wie 2-Propinyl, 2-Butinyl, 3-Butinyl, 1-Methyl-2-propinyl, 2-Pentinyl, 3-Pentinyl, 4-Pentinyl, 1-Methyl-3-butinyl, 2-Methyl-3-butinyl, 1-Methyl-2-butinyl, 1,1-Dimethyl-2-propinyl, 1-Ethyl-2-propinyl, 2-Hexinyl, 3-Hexinyl, 4-Alkinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,2-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl, insbesondere 2-Propinyl, 2-Butinyl und 3-Butinyl, wobei diese Gruppen partiell oder vollständig halogeniert sein können,

d.h. die Wasserstoffatome dieser Gruppen können teilweise oder vollständig durch Halogenatome wie Fluor, Chlor und Brom, insbesondere Fluor und Chlor ersetzt sein, beispielsweise 3-Chlor-2-propinyl, 3-Chlor-2-butinyl und 4-Chlor-3-butinyl;

$C_3$-$C_6$-Alkinyloxy wie 2-Propinyloxy, 2-Butinyloxy, 3-Butinyloxy, 1-Methyl-2-propinyloxy, 2-Pentinyloxy, 3-Pentinyloxy, 3-Pentinyloxy, 4-Pentinyloxy, 1-Methyl-3-butinyloxy, 2-Methyl-3-butinyloxy, 1-Methyl-2-butinyloxy, 1,1-Dimethyl-2-propionyloxy, 1-Ethyl-2-propinyloxy, 2-Hexinyloxy, 3-Hexinyloxy, 4-Alkinyloxy, 5-Hexinyloxy, 1-Methyl-2-pentinyloxy, 1-Methyl-3-pentinyloxy, 1-Methyl-4-pentinyloxy, 2-Methyl-3-pentinyloxy, 2-Methyl-4-pentinyloxy, 3-Methyl-4-pentinyloxy, 4-Methyl-3-pentinyloxy, 1,1-Dimethyl-2-butinyloxy, 1,1-Dimethyl-3-butinyloxy, 1,2-Dimethyl-3-butinyloxy, 2,2-Dimethyl-3-butinyloxy, 1-Ethyl-2-butinyloxy, 1-Ethyl-3-butinyloxy, 2-Ethyl-3-butinyloxy und 1-Ethyl-1-methyl-2-propinyloxy, vorzugsweise 2-Propinyloxy, 2-Butinyloxy, 1-Methyl-2-propinyloxy und 1-Methyl-2-butinyloxy, 2-Propinyloxy, 2-Butinyloxy, 3-Butinyloxy und 1-Methyl-2-propinyloxy; wobei diese Gruppen partiell oder vollständig halogeniert sein können, d.h. die Wasserstoffatome dieser Gruppen können teilweise oder vollständig durch Halogenatome wie Fluor, Chlor und Brom, insbesondere Fluor und Chlor ersetzt sein, beispielsweise 3-Chlor-2-propinyloxy, 3-Chlor-2-butinyloxy und 4-Chlor-3-butinyloxy;

$C_3$-$C_7$-Cycloalkyl wie Cyclopropyl, Cyclobutyl, Cyclopentyl, Cyclohexyl und Cycloheptyl, wobei diese Ringe ein bis 3 $C_1$-$C_4$-Alkylgruppen wie Methyl, Ethyl, Propyl, 1-Methylethyl, Butyl, 1-Methylpropyl, 2-Methylpropyl und 1,1-Dimethylethyl tragen können;

$C_4$-$C_7$-Cycloalkenyl wie Cyclobutenyl, Cyclopentenyl, Cyclohexenyl und Cycloheptenyl, wobei diese Ringe ein bis 3 $C_1$-$C_4$-Alkylgruppen wie Methyl, Ethyl, Propyl, 1-Methylethyl, Butyl, 1-Methylpropyl, 2-Methylpropyl und 1,1-Dimethylethyl tragen können;

$C_3$-$C_7$-Cycloalkyloxy wie Cyclopropyloxy, Cyclobutyloxy, Cyclopentyloxy, Cyclohexyloxy und Cycloheptyloxy, wobei diese Ringe ein bis 3 $C_1$-$C_4$-Alkylgruppen wie Methyl, Ethyl, Propyl, 1-Methylethyl, Butyl, 1-Methylpropyl, 2-Methylpropyl und 1,1-Dimethylethyl tragen können;

oder $C_4$-$C_7$-Cycloalkenyloxy wie 1-Cyclobutenyloxy, 2-Cyclobutenyloxy, 1-Cyclopentenyloxy, 2-Cyclopentenyloxy, 3-Cyclopentenyloxy, 1-Cyclohexenyloxy, 2-Cyclohexenyloxy, 3-Cyclohexenyloxy, 1-Cycloheptenyloxy, 2-Cycloheptenyloxy, 3-Cycloheptenyloxy und 4-Cycloheptenyloxy, wobei diese Ringe ein bis 3 $C_1$-$C_4$-Alkylgruppen wie Methyl, Ethyl, Propyl, 1-Methylethyl, Butyl, 1-Methylpropyl, 2-Methylpropyl und 1,1-Dimethylethyl tragen können;

Phenyl, welches ein bis fünf Halogenatome wie Fluor, Chlor, Brom und Jod, insbesondere Fluor, Chlor und Brom, und/oder ein bis drei der folgenden Reste tragen kann:

$C_1$-$C_4$-Alkyl wie vorstehend genannt;

$C_1$-$C_4$-Halogenalkyl wie vorstehend genannt;

$C_1$-$C_4$-Alkoxy wie vorstehend genannt;

$C_1$-$C_4$-Halogenalkoxy wie vorstehend genannt;

$C_1$-$C_4$-Alkylthio wie Methylthio, Ethylthio, Propylthio, 1-Methylethylthio, Butylthio, 1-Methylpropylthio, 2-Methylpropylthio und 1,1-Dimethylethylthio;

oder $C_1$-$C_4$-Halogenalkylthio, besonders $C_1$-$C_2$-Halogenalkylthio wie Chlormethylthio, Dichlormethylthio, Trichlormethylthio, Fluormethylthio, Difluormethylthio, Trifluormethylthio, Chlorfluormethylthio, Dichlorfluormethylthio, Chlordifluormethylthio, 1-Fluorethylthio, 2-Fluorethylthio, 2,2-Difluorethylthio, 2,2,2-Trifluorethylthio, 2-Chlor-2-fluorethylthio, 2-Chlor-2,2-difluorethylthio, 2,2-Dichlor-2-fluorethylthio, 2,2,2-Trichlorethylthio und Pentafluorethylthio;

A steht für einen cyclischen Rest aus der Gruppe der Formeln A1 bis A7:

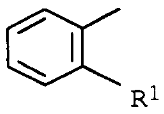 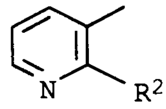 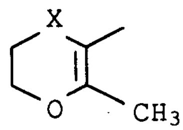 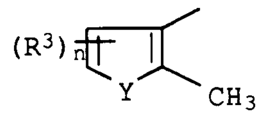

A1 A2 A3 A4

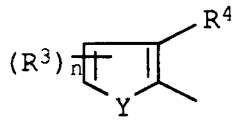 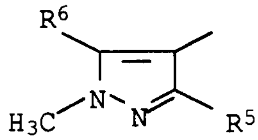 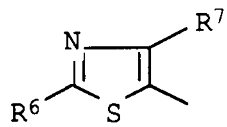

A5 A6 A7

in denen die Substituenten die folgende Bedeutung haben:

X — $-CH_2-$, -S-, -SO- oder $-SO_2-$;

Y — -O- oder -S-;

$R^1$, $R^2$, $R^4$, $R_5$ und $R^7$ — unabhängig voneinander Halogen wie Fluor, Chlor und Brom, $C_1$-$C_4$-Alkyl wie vorstehend genannt, oder $C_1$-$C_4$-Halogenalkyl wie vorstehend genannt;

$R^3$ und $R^6$ — unabhängig voneinander Wasserstoff, Halogen wie Fluor, Chlor und Brom oder $C_1$-$C_4$-Alkyl wie vorstehend genannt;

n — 1 oder 2, wobei die Reste $R^3$ verschieden sein können, wenn der Wert von n 2 beträgt.

Im Hinblick auf die biologische Wirkung besonders bevorzugte Verbindungen der Formel I sind solche, in denen R die vorstehend gegebene Bedeutung hat und A für einen cyclischen Rest aus der Gruppe der Formeln A1 bis A7 steht, wobei X und Y die vorstehend gegebene Bedeutung und die Substituenten für die folgenden Reste stehen:

$R^1$ Halogen wie Fluor, Chlor und Brom Methyl oder $C_1$-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl;

$R^2$ Halogen wie Fluor, Chlor und Brom oder $C_1$-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl;

$R^3$ Wasserstoff oder Methyl;

n 1 oder 2, wobei die Reste $R^3$ verschieden sein können, wenn der Wert von n 2 beträgt;

$R^4$ Halogen wie Fluor, Chlor und Brom oder Methyl;

$R^5$ Methyl oder $C_1$-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl;

$R^6$ Wasserstoff, Halogen wie Fluor, Chlor und Brom oder Methyl;

$R^7$ Halogen wie Fluor, Chlor und Brom Methyl oder $C_1$-Halogenalkyl wie Chlormethyl, Dichlormethyl, Trichlormethyl, Fluormethyl, Difluormethyl, Trifluormethyl, Chlorfluormethyl, Dichlorfluormethyl und Chlordifluormethyl.

Insbesondere sind solche Verbindungen der Formel I bevorzugt, in denen der R die vorstehend gegebene Bedeutung hat und A für einen cyclischen Rest aus der Gruppe der Formeln A1 bis A7 steht, wobei X und Y die vorstehend gegebene Bedeutung und die Substituenten für die folgenden Gruppen stehen:

$R^1$ Chlor, Brom, Jod, Methyl oder Trifluormethyl;

$R^2$ Chlor oder Trifluormethyl;

$R^3$ Wasserstoff oder Methyl;

n 1 oder 2, wobei die Reste $R^3$ verschieden sein können, wenn der Wert von n 2 beträgt;

$R^4$ Chlor oder Methyl;

$R^5$ Methyl, Difluormethyl oder Trifluormethyl;

$R^6$ Wasserstoff, Chlor oder Methyl;

$R^7$ Chlor, Methyl oder Trifluormethyl.

Besonders bevorzugte Verbindungen der Formel I sind in den folgenden Tabellen A bis G zusammengestellt.

Tabelle A

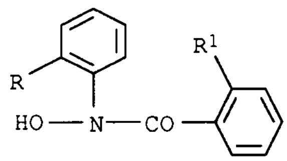

I.1

| $R^1$ | R |
|---|---|
| $CF_3$ | i-$C_3H_7$ |
| $CF_3$ | n-$C_3H_7$ |
| $CF_3$ | n-$C_4H_9$ |
| $CF_3$ | sec.-$C_4H_9$ |
| $CF_3$ | i-$C_4H_9$ |
| $CF_3$ | tert.-$C_4H_9$ |
| $CF_3$ | n-$C_5H_{11}$ |
| $CF_3$ | sec-$C_5H_{11}$ |
| $CF_3$ | n-$C_6H_{13}$ |
| $CF_3$ | n-$C_7H_{15}$ |
| $CF_3$ | sec.-$C_7H_{15}$ |
| $CF_3$ | 1-Methylvinyl |
| $CF_3$ | 2-Methylvinyl |
| $CF_3$ | Allyl |
| $CF_3$ | 2-Methylallyl |
| $CF_3$ | 2-Ethylallyl |
| $CF_3$ | 1-Methylallyl |
| $CF_3$ | 1-Ethylallyl |
| $CF_3$ | 1-Methyl-2-butenyl |
| $CF_3$ | 1-Ethyl-2-butenyl |
| $CF_3$ | 1-Isopropyl-2-butenyl |
| $CF_3$ | 1-n-Butyl-2-butenyl |
| $CF_3$ | 1-Methyl-2-pentenyl |
| $CF_3$ | 1,4-Dimethyl-2-pentenyl |
| $CF_3$ | Propargyl |
| $CF_3$ | 2-Butinyl |
| $CF_3$ | 3-Butinyl |
| $CF_3$ | Ethoxy |
| $CF_3$ | Propoxy |
| $CF_3$ | 1-Methylethoxy |
| $CF_3$ | n-Butoxy |

| $R^1$ | R |
|---|---|
| $CF_3$ | 1-Methylpropoxy |
| $CF_3$ | 2-Methylpropoxy |
| $CF_3$ | 1,1-Dimethylethoxy |
| $CF_3$ | n-Pentyloxy |
| $CF_3$ | n-Hexyloxy |
| $CF_3$ | 2-Ethylhexyloxy |
| $CF_3$ | 2-Propenyloxy |
| $CF_3$ | 2-Butenyloxy |
| $CF_3$ | 2-Methyl-2-propenyloxy |
| $CF_3$ | 2-Pentenyloxy |
| $CF_3$ | 3-Pentenyloxy |
| $CF_3$ | 3-Chlor-2-propenyloxy |
| $CF_3$ | 2,3-Dichlor-2-propenyloxy |
| $CF_3$ | 2,3,3-Trichlor-propenyloxy |
| $CF_3$ | 2-Propinyloxy |
| $CF_3$ | 2-Butinyl-oxy |
| $CF_3$ | 3-Butinyl-oxy |
| $CF_3$ | 1-Methyl-2-propinyloxy |
| $CF_3$ | Cyclopropyl |
| $CF_3$ | Cyclobutyl |
| $CF_3$ | Cyclopentyl |
| $CF_3$ | Cyclohexyl |
| $CF_3$ | 2-Cyclopentenyl |
| $CF_3$ | 1-Cyclopentenyl |
| $CF_3$ | 2-Cyclohexenyl |
| $CF_3$ | 1-Cyclohexenyl |
| $CF_3$ | Cyclopentyloxy |
| $CF_3$ | Cyclohexyloxy |
| $CF_3$ | 2-Cyclopentenyloxy |
| $CF_3$ | 2-Cyclohexenyloxy |
| $CF_3$ | Phenyl |
| Cl | i-$C_3H_7$ |
| Cl | n-$C_3H_7$ |
| Cl | n-$C_4H_9$ |
| Cl | sec.-$C_4H_9$ |
| Cl | i-$C_4H_9$ |
| Cl | tert.-$C_4H_9$ |
| Cl | n-$C_5H_{11}$ |

| $R^1$ | R |
|---|---|
| Cl | sec.-$C_5H_{11}$ |
| Cl | n-$C_6H_{13}$ |
| Cl | n-$C_7H_{15}$ |
| Cl | sec.-$C_7H_{15}$ |
| Cl | 1-Methylvinyl |
| Cl | 2-Methylvinyl |
| Cl | Allyl |
| Cl | 2-Methylvinyl |
| Cl | 2-Ethylallyl |
| Cl | 1-Methylallyl |
| Cl | 1-Ethylallyl |
| Cl | 1-Methyl-2-butenyl |
| Cl | 1-Ethyl-2-butenyl |
| Cl | 1-Isopropyl-2-butenyl |
| Cl | 1-n-Butyl-2-butenyl |
| Cl | Methyl-2-pentenyl |
| Cl | 1,4-Dimethyl-2-pentenyl |
| Cl | Propargyl |
| Cl | 2-Butinyl |
| Cl | 3-Butinyl |
| Cl | Ethoxy |
| Cl | Propoxy |
| Cl | 1-Methylethoxy |
| Cl | n-Butoxy |
| Cl | 1-Methylpropoxy |
| Cl | 2-Methylpropoxy |
| Cl | 1,1-Dimethylethoxy |
| Cl | n-Pentyloxy |
| Cl | n-Hexyloxy |
| Cl | 2-Ethylhexyloxy |
| Cl | 2-Propenyloxy |
| Cl | 2-Butenyloxy |
| Cl | 2-Methyl-2-propenyloxy |
| Cl | 2-Pentenyloxy |
| Cl | 3-Pentenyloxy |
| Cl | 3-Chlor-2-propenyloxy |
| Cl | 2,3-Dichlor-2-propenyloxy |
| Cl | 2,3,3-Trichlor-propenyloxy |

| $R^1$ | R |
|---|---|
| Cl | 2-Propinyloxy |
| Cl | 2-Butinyl-oxy |
| Cl | 3-Butinyl-oxy |
| Cl | 1-Methyl-2-propinyloxy |
| Cl | Cyclopropyl |
| Cl | Cyclobutyl |
| Cl | Cyclopentyl |
| Cl | Cyclohexyl |
| Cl | 2-Cyclopentenyl |
| Cl | 1-Cyclopentenyl |
| Cl | 2-Cyclohexenyl |
| Cl | 1-Cyclohexenyl |
| Cl | Cyclopentyloxy |
| Cl | Cyclohexyloxy |
| Cl | 2-Cyclopentenyloxy |
| Cl | 2-Cyclohexenyloxy |
| Cl | Phenyl |

Tabelle B

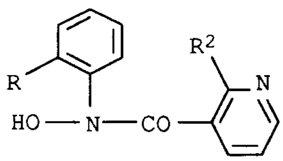

I.2

| $R^2$ | R |
|---|---|
| Cl | i-$C_3H_7$ |
| Cl | n-$C_3H_7$ |
| Cl | n-$C_4H_9$ |
| Cl | sec.-$C_4H_9$ |
| Cl | i-$C_4H_9$ |
| Cl | tert.-$C_4H_9$ |
| Cl | n-$C_5H_{11}$ |
| Cl | sec.-$C_5H_{11}$ |
| Cl | n-$C_6H_{13}$ |
| Cl | n-$C_7H_{15}$ |
| Cl | sec.-$C_7H_{15}$ |
| Cl | 1-Methylvinyl |
| Cl | 2-Methylvinyl |
| Cl | Allyl |
| Cl | 2-Methylallyl |
| Cl | 2-Ethylallyl |
| Cl | 1-Methylallyl |
| Cl | 1-Ethylallyl |
| Cl | 1-Methyl-2-butenyl |
| Cl | 1-Ethyl-2-butenyl |
| Cl | 1-Isopropyl-2-butenyl |
| Cl | 1-n-Butyl-2-butenyl |
| Cl | 1-Methyl-2-pentenyl |
| Cl | 1,4-Dimethyl-2-pentenyl |
| Cl | Propargyl |
| Cl | 2-Butinyl |
| Cl | 3-Butinyl |
| Cl | Ethoxy |
| Cl | Propoxy |
| Cl | 1-Methylethoxy |
| Cl | n-Butoxy |

| $R^2$ | R |
|---|---|
| Cl | 1-Methylpropoxy |
| Cl | 2-Methylpropoxy |
| Cl | 1,1-Dimethylethoxy |
| Cl | n-Pentyloxy |
| Cl | n-Hexyloxy |
| Cl | 2-Ethylhexyloxy |
| Cl | 2-Propenyloxy |
| Cl | 2-Butenyloxy |
| Cl | 2-Methyl-2-propenyloxy |
| Cl | 2-Pentenyloxy |
| Cl | 3-Pentenyloxy |
| Cl | 3-Chlor-2-propenyloxy |
| Cl | 2,3-Dichlor-2-propenyloxy |
| Cl | 2,3,3-Trichlor-propenyloxy |
| Cl | 2-Propinyloxy |
| Cl | 2-Butinyl-oxy |
| Cl | 3-Butinyl-oxy |
| Cl | 1-Methyl-2-propinyloxy |
| Cl | Cyclopropyl |
| Cl | Cyclobutyl |
| Cl | Cyclopentyl |
| Cl | Cyclohexyl |
| Cl | 2-Cyclopentenyl |
| Cl | 1-Cyclopentenyl |
| Cl | 2-Cyclohexenyl |
| Cl | 1-Cyclohexenyl |
| Cl | Cyclopentyloxy |
| Cl | Cyclohexyloxy |
| Cl | 2-Cyclopentenyloxy |
| Cl | 2-Cyclohexenyloxy |
| Cl | i-$C_3H_7$ |
| Cl | n-$C_3H_7$ |
| Cl | n-$C_4H_9$ |
| Cl | sec.-$C_4H_9$ |
| Cl | i-$C_4H_9$ |
| Cl | tert.-$C_4H_9$ |
| Cl | n-$C_5H_{11}$ |
| Cl | sec.-$C_5H_{11}$ |

| $R^2$ | R |
|---|---|
| Cl | $n-C_6H_{13}$ |
| Cl | $n-C_7H_{15}$ |
| Cl | sec.-$C_7H_{15}$ |
| Cl | Ethoxy |
| Cl | Propoxy |
| Cl | 1-Methylethoxy |
| Cl | n-Butoxy |
| Cl | 1-Methylpropoxy |
| Cl | 2-Methylpropoxy |
| Cl | 1,1-Dimethylethoxy |
| Cl | n-Pentyloxy |
| Cl | n-Hexyloxy |
| Cl | Cyclopentyl |
| Cl | Phenyl |

Tabelle C

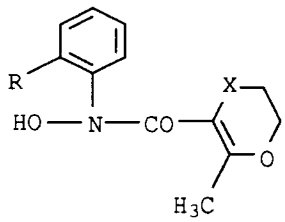

I.3

| X | R |
|---|---|
| $CH_2$ | i-$C_3H_7$ |
| $CH_2$ | n-$C_3H_7$ |
| $CH_2$ | n-$C_4H_9$ |
| $CH_2$ | sec.-$C_4H_9$ |
| $CH_2$ | i-$C_4H_9$ |
| $CH_2$ | tert.-$C_4H_9$ |
| $CH_2$ | n-$C_5H_{11}$ |
| $CH_2$ | sec.-$C_5H_{11}$ |
| $CH_2$ | n-$C_6H_{13}$ |
| $CH_2$ | n-$C_7H_{15}$ |
| $CH_2$ | sec.-$C_7H_{15}$ |
| $CH_2$ | 1-Methylvinyl |
| $CH_2$ | 2-Methylvinyl |
| $CH_2$ | Allyl |
| $CH_2$ | 2-Methylallyl |
| $CH_2$ | 2-Ethylallyl |
| $CH_2$ | 1-Methylallyl |
| $CH_2$ | 1-Ethylallyl |
| $CH_2$ | 1-Methyl-2-butenyl |
| $CH_2$ | 1-Ethyl-2-butenyl |
| $CH_2$ | 1-Isopropyl-2-butenyl |
| $CH_2$ | 1-n-Butyl-2-butenyl |
| $CH_2$ | 1-Methyl-2-pentenyl |
| $CH_2$ | 1,4-Dimethyl-2-pentenyl |
| $CH_2$ | Propargyl |
| $CH_2$ | 2-Butinyl |
| $CH_2$ | 3-Butinyl |
| $CH_2$ | Ethoxy |
| $CH_2$ | Propoxy |

| X | R |
|---|---|
| $CH_2$ | 1-Methylethoxy |
| $CH_2$ | n-Butoxy |
| $CH_2$ | 1-Methylpropoxy |
| $CH_2$ | 2-Methylpropoxy |
| $CH_2$ | 1,1-Dimethylethoxy |
| $CH_2$ | n-Pentyloxy |
| $CH_2$ | n-Hexyloxy |
| $CH_2$ | 2-Ethylhexyloxy |
| $CH_2$ | 2-Propenyloxy |
| $CH_2$ | 2-Butenyloxy |
| $CH_2$ | 2-Methyl-2-propenyloxy |
| $CH_2$ | 2-Pentenyloxy |
| $CH_2$ | 3-Pentenyloxy |
| $CH_2$ | 3-Chlor-2-propenyloxy |
| $CH_2$ | 2,3-Dichlor-2-propenyloxy |
| $CH_2$ | 2,3,3-Trichlor-propenyloxy |
| $CH_2$ | 2-Propinyloxy |
| $CH_2$ | 2-Butinyl-oxy |
| $CH_2$ | 3-Butinyl-oxy |
| $CH_2$ | 1-Methyl-2-propinyloxy |
| $CH_2$ | Cyclopropyl |
| $CH_2$ | Cyclobutyl |
| $CH_2$ | Cyclopentyl |
| $CH_2$ | Cyclohexyl |
| $CH_2$ | 2-Cyclopentenyl |
| $CH_2$ | 1-Cyclopentenyl |
| $CH_2$ | 2-Cyclohexenyl |
| $CH_2$ | 1-Cyclohexenyl |
| $CH_2$ | Cyclopentyloxy |
| $CH_2$ | Cyclohexyloxy |
| $CH_2$ | 2-Cyclopentenyloxy |
| $CH_2$ | 2-Cyclohexenyloxy |
| S | i-$C_3H_7$ |
| S | n-$C_3H_7$ |
| S | n-$C_4H_9$ |
| S | sec.-$C_4H_9$ |
| S | i-$C_4H_9$ |
| S | tert.-$C_4H_9$ |

| X | R |
|---|---|
| S | n-$C_5H_{11}$ |
| S | sec.-$C_5H_{11}$ |
| S | n-$C_6H_{13}$ |
| S | n-$C_7H_{15}$ |
| S | sec.-$C_7H_{15}$ |
| S | Ethoxy |
| S | Propoxy |
| S | 1-Methylethoxy |
| S | n-Butoxy |
| S | 1-Methylpropoxy |
| S | 2-Methylpropoxy |
| S | 1,1-Dimethylethoxy |
| S | n-Pentyloxy |
| S | n-Hexyloxy |
| S | Cyclopentyl |
| S | Phenyl |

Tabelle D

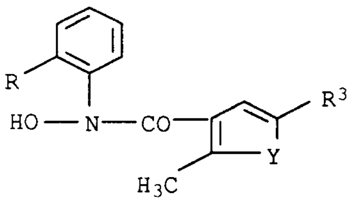

I.4

| $R^3$ | R | Y |
|---|---|---|
| H | i-$C_3H_7$ | O |
| H | n-$C_3H_7$ | O |
| H | n-$C_4H_9$ | O |
| H | sec.-$C_4H_9$ | O |
| H | i-$C_4H_9$ | O |
| H | tert.-$C_4H_9$ | O |
| H | n-$C_5H_{11}$ | O |
| H | sec.-$C_5H_{11}$ | O |
| H | n-$C_6H_{13}$ | O |
| H | n-$C_7H_{15}$ | O |
| H | sec.-$C_7H_{15}$ | O |
| H | Ethoxy | O |
| H | Propoxy | O |
| H | 1-Methylethoxy | O |
| H | n-Butoxy | O |
| H | 1-Methylpropoxy | O |
| H | 2-Methylpropoxy | O |
| H | 1,1-Dimethylethoxy | O |
| H | n-Pentyloxy | O |
| H | n-Hexyloxy | O |
| H | Cyclopentyl | O |
| H | Cyclohexyl | O |
| H | 2-Cyclopentenyl | O |
| H | 1-Cyclopentenyl | O |
| H | 2-Cyclohexenyl | O |
| H | 1-Cyclohexenyl | O |
| H | Cyclopentyloxy | O |
| H | Cyclohexyloxy | O |
| H | 2-Cyclopentenyloxy | O |

| $R^3$ | R | Y |
|---|---|---|
| H | 2-Cyclohexenyloxy | O |
| $CH_3$ | i-$C_3H_7$ | O |
| $CH_3$ | n-$C_3H_7$ | O |
| $CH_3$ | n-$C_4H_9$ | O |
| $CH_3$ | sec.-$C_4H_9$ | O |
| $CH_3$ | i-$C_4H_9$ | O |
| $CH_3$ | tert.-$C_4H_9$ | O |
| $CH_3$ | n-$C_5H_{11}$ | O |
| $CH_3$ | sec.-$C_5H_{11}$ | O |
| $CH_3$ | n-$C_6H_{13}$ | O |
| $CH_3$ | n-$C_7H_{15}$ | O |
| $CH_3$ | sec.-$C_7H_{15}$ | O |
| $CH_3$ | Ethoxy | O |
| $CH_3$ | Propoxy | O |
| $CH_3$ | 1-Methylethoxy | O |
| $CH_3$ | n-Butoxy | O |
| $CH_3$ | 1-Methylpropoxy | O |
| $CH_3$ | 2-Methylpropoxy | O |
| $CH_3$ | 1,1-Dimethylethoxy | O |
| $CH_3$ | n-Pentyloxy | O |
| $CH_3$ | n-Hexyloxy | O |
| $CH_3$ | Cyclopentyl | O |
| $CH_3$ | Phenyl | O |

Tabelle E

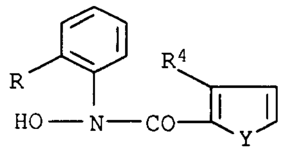

I.5

| $R^4$ | R | Y |
| --- | --- | --- |
| $CH_3$ | $i-C_3H_7$ | O |
| $CH_3$ | $n-C_3H_7$ | O |
| $CH_3$ | $n-C_4H_9$ | O |
| $CH_3$ | sec.-$C_4H_9$ | O |
| $CH_3$ | $i-C_4H_9$ | O |
| $CH_3$ | tert.-$C_4H_9$ | O |
| $CH_3$ | $n-C_5H_{11}$ | O |
| $CH_3$ | sec.-$C_5H_{11}$ | O |
| $CH_3$ | $n-C_6H_{13}$ | O |
| $CH_3$ | $n-C_7H_{15}$ | O |
| $CH_3$ | sec.-$C_7H_{15}$ | O |
| $CH_3$ | Ethoxy | O |
| $CH_3$ | Propoxy | O |
| $CH_3$ | 1-Methylethoxy | O |
| $CH_3$ | n-Butoxy | O |
| $CH_3$ | 1-Methylpropoxy | O |
| $CH_3$ | 2-Methylpropoxy | O |
| $CH_3$ | 1,1-Dimethylethoxy | O |
| $CH_3$ | n-Pentyloxy | O |
| $CH_3$ | n-Hexyloxy | O |
| $CH_3$ | Cyclopentyl | O |
| $CH_3$ | Cyclopentenyl | O |
| $CH_3$ | $i-C_3H_7$ | S |
| $CH_3$ | $n-C_3H_7$ | S |
| $CH_3$ | $n-C_4H_9$ | S |
| $CH_3$ | sec.-$C_4H_9$ | S |
| $CH_3$ | $i-C_4H_9$ | S |
| $CH_3$ | tert.-$C_4H_9$ | S |
| $CH_3$ | $n-C_5H_{11}$ | S |
| $CH_3$ | sec.-$C_5H_{11}$ | S |
| $CH_3$ | $n-C_6H_{13}$ | S |

| $R^4$ | R | Y |
|---|---|---|
| $CH_3$ | n-$C_7H_{15}$ | S |
| $CH_3$ | sec.-$C_7H_{15}$ | S |
| $CH_3$ | Ethoxy | S |
| $CH_3$ | Propoxy | S |
| $CH_3$ | 1-Methylethoxy | S |
| $CH_3$ | n-Butoxy | S |
| $CH_3$ | 1-Methylpropoxy | S |
| $CH_3$ | 2-Methylpropoxy | S |
| $CH_3$ | 1,1-Dimethylethoxy | S |
| $CH_3$ | n-Pentyloxy | S |
| $CH_3$ | n-Hexyloxy | S |
| $CH_3$ | Cyclopentyl | S |
| $CH_3$ | Cyclopentenyl | S |

Tabelle F

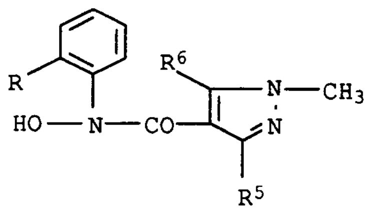

I.6

| $R^5$ | $R^6$ | R |
|---|---|---|
| $CH_3$ | H | i-$C_3H_7$ |
| $CH_3$ | H | n-$C_3H_7$ |
| $CH_3$ | H | n-$C_4H_9$ |
| $CH_3$ | H | sec.-$C_4H_9$ |
| $CH_3$ | H | i-$C_4H_9$ |
| $CH_3$ | H | tert.-$C_4H_9$ |
| $CH_3$ | H | n-$C_5H_{11}$ |
| $CH_3$ | H | sec.-$C_5H_{11}$ |
| $CH_3$ | H | n-$C_6H_{13}$ |
| $CH_3$ | H | n-$C_7H_{15}$ |
| $CH_3$ | H | sec.-$C_7H_{15}$ |
| $CH_3$ | H | 1-Methylvinyl |
| $CH_3$ | H | 2-Methylvinyl |
| $CH_3$ | H | Allyl |
| $CH_3$ | H | 2-Methylallyl |
| $CH_3$ | H | 2-Ethylallyl |
| $CH_3$ | H | 1-Methylallyl |
| $CH_3$ | H | 1-Ethylallyl |
| $CH_3$ | H | 1-Methyl-2-butenyl |
| $CH_3$ | H | 1-Ethyl-2-butenyl |
| $CH_3$ | H | 1-Isopropyl-2-butenyl |
| $CH_3$ | H | 1-n-Butyl-2-butenyl |
| $CH_3$ | H | 1-Methyl-2-pentenyl |
| $CH_3$ | H | 1,4-Dimethyl-2-pentenyl |
| $CH_3$ | H | Propargyl |
| $CH_3$ | H | 2-Butinyl |
| $CH_3$ | H | 3-Butinyl |
| $CH_3$ | H | Ethoxy |
| $CH_3$ | H | Propoxy |

| $R^5$ | $R^6$ | R |
|---|---|---|
| $CH_3$ | H | 1-Methylethoxy |
| $CH_3$ | H | n-Butoxy |
| $CH_3$ | H | 1-Methylpropoxy |
| $CH_3$ | H | 2-Methylpropoxy |
| $CH_3$ | H | 1,1-Dimethylethoxy |
| $CH_3$ | H | n-Pentyloxy |
| $CH_3$ | H | n-Hexyloxy |
| $CH_3$ | H | 2-Ethylhexyloxy |
| $CH_3$ | H | 2-Propenyloxy |
| $CH_3$ | H | 2-Butenyloxy |
| $CH_3$ | H | 2-Methyl-2-propenyloxy |
| $CH_3$ | H | 2-Pentenyloxy |
| $CH_3$ | H | 3-Pentenyloxy |
| $CH_3$ | H | 3-Chlor-2-propenyloxy |
| $CH_3$ | H | 2,3-Dichlor-2-propenyloxy |
| $CH_3$ | H | 2,3,3-Trichlor-propenyloxy |
| $CH_3$ | H | 2-Propinyloxy |
| $CH_3$ | H | 2-Butinyl-oxy |
| $CH_3$ | H | 3-Butinyl-oxy |
| $CH_3$ | H | 1-Methyl-2-propinyloxy |
| $CH_3$ | H | Cyclopropyl |
| $CH_3$ | H | Cyclobutyl |
| $CH_3$ | H | Cyclopentyl |
| $CH_3$ | H | Cyclohexyl |
| $CH_3$ | H | 2-Cyclopentenyl |
| $CH_3$ | H | 1-Cyclopentenyl |
| $CH_3$ | H | 2-Cyclohexenyl |
| $CH_3$ | H | 1-Cyclohexenyl |
| $CH_3$ | H | Cyclopentyloxy |
| $CH_3$ | H | Cyclohexyloxy |
| $CH_3$ | H | 2-Cyclopentenyloxy |
| $CH_3$ | H | 2-Cyclohexenyloxy |
| $CF_3$ | H | i-$C_3H_7$ |
| $CF_3$ | H | n-$C_3H_7$ |
| $CF_3$ | H | n-$C_4H_9$ |
| $CF_3$ | H | sec.-$C_4H_9$ |
| $CF_3$ | H | i-$C_4H_9$ |
| $CF_3$ | H | tert.-$C_4H_9$ |

| $R^5$ | $R^6$ | R |
|---|---|---|
| $CF_3$ | H | n-$C_5H_{11}$ |
| $CF_3$ | H | sec.-$C_5H_{11}$ |
| $CF_3$ | H | n-$C_6H_{13}$ |
| $CF_3$ | H | n-$C_7H_{15}$ |
| $CF_3$ | H | sec.-$C_7H_{15}$ |
| $CF_3$ | H | Ethoxy |
| $CF_3$ | H | Propoxy |
| $CF_3$ | H | 1-Methylethoxy |
| $CF_3$ | H | n-Butoxy |
| $CF_3$ | H | 1-Methylpropoxy |
| $CF_3$ | H | 2-Methylpropoxy |
| $CF_3$ | H | 1,1-Dimethylethoxy |
| $CF_3$ | H | n-Pentyloxy |
| $CF_3$ | H | n-Hexyloxy |
| $CF_3$ | H | Cyclopentyl |
| $CF_3$ | H | Cyclopentenyl |
| $CF_3$ | H | Phenyl |

Tabelle G

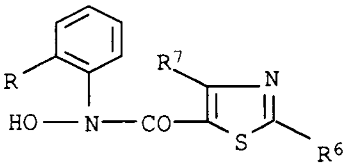

I.7

| $R^7$ | $R^6$ | R |
|---|---|---|
| $CF_3$ | $CH_3$ | i-$C_3H_7$ |
| $CF_3$ | $CH_3$ | n-$C_3H_7$ |
| $CF_3$ | $CH_3$ | n-$C_4H_9$ |
| $CF_3$ | $CH_3$ | sec.-$C_4H_9$ |
| $CF_3$ | $CH_3$ | i-$C_4H_9$ |
| $CF_3$ | $CH_3$ | tert.-$C_4H_9$ |
| $CF_3$ | $CH_3$ | n-$C_5H_{11}$ |
| $CF_3$ | $CH_3$ | sec.-$C_5H_{11}$ |
| $CF_3$ | $CH_3$ | n-$C_6H_{13}$ |
| $CF_3$ | $CH_3$ | n-$C_7H_{15}$ |
| $CF_3$ | $CH_3$ | sec.-$C_7H_{15}$ |
| $CF_3$ | $CH_3$ | 1-Methylvinyl |
| $CF_3$ | $CH_3$ | 2-Methylvinyl |
| $CF_3$ | $CH_3$ | Allyl |
| $CF_3$ | $CH_3$ | 2-Methylallyl |
| $CF_3$ | $CH_3$ | 2-Ethylallyl |
| $CF_3$ | $CH_3$ | 1-Methylallyl |
| $CF_3$ | $CH_3$ | 1-Ethylallyl |
| $CF_3$ | $CH_3$ | 1-Methyl-2-butenyl |
| $CF_3$ | $CH_3$ | 1-Ethyl-2-butenyl |
| $CF_3$ | $CH_3$ | 1-Isopropyl-2-butenyl |
| $CF_3$ | $CH_3$ | 1-n-Butyl-2-butenyl |
| $CF_3$ | $CH_3$ | 1-Methyl-2-pentenyl |
| $CF_3$ | $CH_3$ | 1,4-Dimethyl-2-pentenyl |
| $CF_3$ | $CH_3$ | Propargyl |
| $CF_3$ | $CH_3$ | 2-Butinyl |
| $CF_3$ | $CH_3$ | 3-Butinyl |
| $CF_3$ | $CH_3$ | Ethoxy |
| $CF_3$ | $CH_3$ | Propoxy |
| $CF_3$ | $CH_3$ | 1-Methylethoxy |

| $R^7$ | $R^6$ | R |
|---|---|---|
| $CF_3$ | $CH_3$ | n-Butoxy |
| $CF_3$ | $CH_3$ | 1-Methylpropoxy |
| $CF_3$ | $CH_3$ | 2-Methylpropoxy |
| $CF_3$ | $CH_3$ | 1,1-Dimethylethoxy |
| $CF_3$ | $CH_3$ | n-Pentyloxy |
| $CF_3$ | $CH_3$ | n-Hexyloxy |
| $CF_3$ | $CH_3$ | 2-Ethylhexyloxy |
| $CF_3$ | $CH_3$ | 2-Propenyloxy |
| $CF_3$ | $CH_3$ | 2-Butenyloxy |
| $CF_3$ | $CH_3$ | 2-Methyl-2-propenyloxy |
| $CF_3$ | $CH_3$ | 2-Pentenyloxy |
| $CF_3$ | $CH_3$ | 3-Pentenyloxy |
| $CF_3$ | $CH_3$ | 3-Chlor-2-propenyloxy |
| $CF_3$ | $CH_3$ | 2,3-Dichlor-2-propenyloxy |
| $CF_3$ | $CH_3$ | 2,3,3-Trichlor-propenyloxy |
| $CF_3$ | $CH_3$ | 2-Propinyloxy |
| $CF_3$ | $CH_3$ | 2-Butinyl-oxy |
| $CF_3$ | $CH_3$ | 3-Butinyl-oxy |
| $CF_3$ | $CH_3$ | 1-Methyl-2-propinyloxy |
| $CF_3$ | $CH_3$ | Cyclopropyl |
| $CF_3$ | $CH_3$ | Cyclobutyl |
| $CF_3$ | $CH_3$ | Cyclopentyl |
| $CF_3$ | $CH_3$ | Cyclohexyl |
| $CF_3$ | $CH_3$ | 2-Cyclopentenyl |
| $CF_3$ | $CH_3$ | 1-Cyclopentenyl |
| $CF_3$ | $CH_3$ | 2-Cyclohexenyl |
| $CF_3$ | $CH_3$ | 1-Cyclohexenyl |
| $CF_3$ | $CH_3$ | Cyclopentyloxy |
| $CF_3$ | $CH_3$ | Cyclohexyloxy |
| $CF_3$ | $CH_3$ | 2-Cyclopentenyloxy |
| $CF_3$ | $CH_3$ | 2-Cyclohexenyloxy |
| $CH_3$ | $CH_3$ | i-$C_3H_7$ |
| $CH_3$ | $CH_3$ | n-$C_3H_7$ |
| $CH_3$ | $CH_3$ | n-$C_4H_9$ |
| $CH_3$ | $CH_3$ | sec.-$C_4H_9$ |
| $CH_3$ | $CH_3$ | i-$C_4H_9$ |
| $CH_3$ | $CH_3$ | tert.-$C_4H_9$ |
| $CH_3$ | $CH_3$ | n-$C_5H_{11}$ |

| $R^7$ | $R^6$ | R |
|---|---|---|
| $CH_3$ | $CH_3$ | sec.-$C_5H_{11}$ |
| $CH_3$ | $CH_3$ | n-$C_6H_{13}$ |
| $CH_3$ | $CH_3$ | n-$C_7H_{15}$ |
| $CH_3$ | $CH_3$ | sec.-$C_7H_{15}$ |
| $CH_3$ | $CH_3$ | Ethoxy |
| $CH_3$ | $CH_3$ | Propoxy |
| $CH_3$ | $CH_3$ | 1-Methylethoxy |
| $CH_3$ | $CH_3$ | n-Butoxy |
| $CH_3$ | $CH_3$ | 1-Methylpropoxy |
| $CH_3$ | $CH_3$ | 2-Methylpropoxy |
| $CH_3$ | $CH_3$ | 1,1-Dimethylethoxy |
| $CH_3$ | $CH_3$ | n-Pentyloxy |
| $CH_3$ | $CH_3$ | n-Hexyloxy |
| $CH_3$ | $CH_3$ | Cyclopentyl |
| $CH_3$ | $CH_3$ | Cyclopentenyl |
| $CH_3$ | $CH_3$ | Phenyl |

Die neuen Wirkstoffe eignen sich besonders zum Schutz von verschiedenen Materialien gegen den Abbau bzw. die Zerstörung durch Bakterien oder Pilze oder gegen den Befall und Bewuchs durch Mikroorganismen. Materialien, die mit den neuen Wirkstoffen konserviert bzw. mikrozid ausgerüstet werden können, sind beispielsweise Leime und Klebstoffe, Stärkelösungen, Wachsemulsionen, Tonemulsionen, Schlichten, Appreturen, Spinnbäder, Gelatinezubereitungen, Fensterkitt, Fugendichtungsmassen, Kühlschmierstoffe, Bohröle, Treibstoffe, Kunststoffdispersionen, Dispersionsfarben, Textilien, Leder, Rohhäute und Kosmetika. Weiterhin sind die Verbindungen als Schleimbekämpfungsmittel in der Papierindustrie, in Rückkühlwerken und in Luftbefeuchtungsanlagen geeignet.

Des weiteren eignen sich die Verbindungen I zum Schutz folgender Pflanzenarten vor dem Befall durch Mikroorganismen:
Getreide (z.B. Weizen, Gerste, Roggen, Hafer, Reis, Sorhum und Verwandte); Rüben (z.B. Zucker- und Futterrüben); Kern-, Stein- und Beerenobst (z.B. Äpfel, Birnen, Pflaumen, Pfirsiche, Mandeln, Kirschen, Erdbeeren, Himbeeren und Brombeeren); Hülsenfrüchte (z.B. Bohnen, Linsen, Erbsen, Soja); Ölkulturen (z.B. Raps, Senf, Mohn, Oliven, Sonnenblumen, Kokos, Rizinus, Kakao, Erdnüsse); Gurkengewächse (z.B. Kürbis, Gurken, Melonen); Fasergewächse (z.B. Baumwolle, Flachs, Hanf, Jute); Citrusfrüchte (z.B. Orangen, Zitronen, Pampelmusen, Mandarinen); Gemüsesorten (z.B. Spinat, Kopfsalat, Spargel, Kohlarten, Möhren, Zwiebeln, Tomaten, Kartoffeln, Paprika); Lorbeergewächse (z.B. Avocado, Cinnamonum, Kampfer) oder Pflanzen wie Mais, Tabak, Nüsse, Kaffee, Zuckerrohr, Tee, Weintrauben, Hopfen, Bananen- und Naturkautschukgewächse. Pflanzen seien im Rahmen vorliegender Erfindung aber auch alle Arten von sonstigen Grünbewachsungen, seien es Zierpflanzen (Compositen), Grasflächen, Böschungen oder allgemeine niedrige Bodenbedeckungen (cover corps).

Folgende Mikroorganismen lassen sich beispielsweise mit den neuen Verbindungen I bekämpfen:
Straphylococcus aureus, Escherichia coli, Klebsielle pneumoniae, Citrobacter freundii, Proteus vulgaris, Pseudomonas aeruginosa, Desulfovibrio desulfuricans, Streptoverticillium rubrireticuli, Aspergillus niger, Aspergillus versicolor, Penicillium funiculosum, Penicillium expansum, Penicillium glaucum, Paecilomyces variotii, Trichoderma viride, Chaetomium globosum, Aspergillus amstelodami, Phoma pigmentovora, Phoma violacea, Aureobasidium pullulans, Saccharomyces cerevisiae, Alternaria tenuis, Stemphylium macrosporoideum, Cladosporium herbarum, Cladosporium resinae, Candida albicans, Trichophyton mentagrophytes, Geotrichum candidans, Monilia sitophila, Scenedesmus quadricauda, Chlorella vulgaris, Nostoc muscorium, Oscillatoria limosa und Anabaena constricta.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanzen gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser, Trägerstoffe wie natürliche Gesteinsmehle, z.B. Kaoline, Tonerden, Talkum, Kreide und synthetische Gesteinsmehle (z.B. hochdisperse Kieselsäure, Silikate), Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die Formulierungen enthalten im allgemeinen zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR/HPLC/GC-Spektrum) eingesetzt.

Als übliche Anwendungskonzentration wählt man - bezogen auf das Gewicht des zu schützenden Materials - 0,001 bis 5 Gew.-%, bevorzugt 0,01 bis 2 Gew.-% an Wirkstoff; beim Einsatz zur Wasserbehandlung, bei der Erdölförderung, in Bohr- und Schneidölen, Treibstoffen, in Schwimmbädern, Rückkühlwerken, Luftbefeuchtungsanlagen oder in der Papierindustrie sind Wirkstoffmengen von 5 bis 500 ppm ausreichend. Gebrauchsfertige Desinfektionsmittellösungen enthalten z.B. 0,5 bis 10 Gew.-% an Wirkstoff.

Beispiele für solche Zubereitungen sind:

I. eine Lösung aus 90 Gew.-Teilen der Verbindung Nr. 3 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 4, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.

III. eine wäßrige Dispersion aus 20 Gew.-Teilen der Verbindung Nr. 1, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.

IV. eine wäßrige Dispersion aus 20 Gew.-Teilen der Verbindung Nr. 3, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;

V. eine in einer Hammermühle vermahlene Mischung aus 80 Gew.-Teilen der Verbindung Nr. 2, 3 Gew.-Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;

VI. eine innige Mischung aus 3 Gew.-Teilen der Verbindung Nr. 1 und 97 Gew.-Teilen feinteiligem Kaolin. Dieses Stäubemittel enthält 3 Gew.-% Wirkstoff;

VII. eine innige Mischung aus 30 Gew.-Teilen der Verbindung Nr. 4, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;

VIII. eine stabile wäßrige Dispersion aus 40 Gew.-Teilen der Verbindung Nr. 2, 10 Gew.-Teilen des Natriumsalzes eines Phenosulfonsäure harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 3, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls;

X. eine in einer Hammermühle vermahlene Mischung aus 10 Gew.-Teilen der Verbindung Nr. 1, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe,

die 0,1 Gew.% des Wirkstoffs enthält.

Die Wirkstoffe wirken für sich allein als schaumarme Biozide. Eine bedeutende Steigerung der Wirkung dieser Verbindungen enthaltender biozider Zubereitungen wird erzielt, wenn man ihnen noch Tri-$C_6$- bis $C_{12}$-alkylmethylammoniumsalze, vorzugsweise in Mengen von 20 bis 40 Gew.-%, bezogen auf das Gewicht der Verbindungen der allgemeinen Formel I, zusetzt.

Die Wirkstoffe können auch mit anderen bekannten Mikrobiziden gemischt werden. In vielen Fällen erhält man dabei einen synergistischen Effekt, d.h. die mikrobizide Wirksamkeit der Mischung ist größer als die der (addierten) Wirksamkeiten der Einzelkomponenten.

Die Zumischung der bekannten Mikrobizide zu den neuen Substanzen kann in einem Gewichtsverhältnis von 1:100 bis 100:1 erfolgen.

Solche Wirkstoffe sind beispielsweise:
2-(Thiocyanomethylthio)-benzthiazol
1-[2-(2,4-Dichlorphenyl)-2-(2-propenyl-oxy)-ethyl]-1H-imidazol
2,4,5,6-Tetrachlor-isophthalodinitril
Methylenbisthiocyanat
Tributylzinnoxid, -naphthenat, -benzoat, -salicylat
Mercaptobenzthiazol
1,2-Benzisothiazolon und seine Alkalisalze
Alkaliverbindungen des N'-Hydroxy-N-cyclohexyl-diazeniumoxids
2-(Methoxy-carbonylamino)-benzimidazol
2-Methyl-3-oxo-5-chlor-thiazolin-3-on
Trihydroxymethyl-nitro-methan
Glutardialdehyd
Chloracetamid
Polyhexamethylenbisguanide
5-Chlor-2-methyl-4-isothiazolin-3-on + Magnesiumsalze
3,5-Dimethyltetrahydro-1,3,5-2H-thiadiazin-2-thion
Hexahydrotriazin
N,N-Methylolchloracetamid
2-n-Octyl-4-isothiazol-in-3-on
Oxazolidine
Bisoxazolidine
2,5-Dihydro-2,5-dialkoxy-2,5-dialkylfurane
Diethyl-dodecyl-benzyl-ammoniumchlorid
Dimethyl-octadecyl-dimethylbenzyl-ammoniumchlorid
Dimethyl-didecyl-ammoniumchlorid
Dimethyl-didodecyl-ammoniumchlorid
Trimethyl-tetradecylammoniumchlorid
Benzyl-dimethyl-alkyl-($C_{12}$-$C_{18}$)-ammoniumchlorid
Dichlorbenzyl-dimethyl-dodecyl-ammoniumchlorid
Cetylpyridiniumchlorid
Cetylpyridiniumbromid
Cetyl-trimethyl-ammoniumchlorid
Laurylpyridiniumchlorid
Laurylpyridiniumbisulfat
Benzyl-dodecyl-di(beta-oxyethyl)-ammoniumchlorid
Dodecylbenzyl-trimethyl-ammoniumchlorid
n-Alkyl-dimethyl-benzyl-ammoniumchlorid
(Alkylrest: 40 % $C_{12}$, 50 % $C_{14}$, 10 % $C_{16}$)
Lauryl-dimethyl-ethyl-ammoniumethylsulfat
n-Alkyl-dimethyl-(1-naphthylmethyl)-ammoniumchlorid
(Alkylrest: 98 % $C_{12}$, 2 % $C_{14}$)
Cetyldimethylbenzylammoniumchlorid
Lauryldimethylbenzylammoniumchlorid

Weitere mögliche Mischungspartner sind beispielsweise:
1,3-Dimethylol-5,5-dimethylhydantoin
Dimethylolharnstoff
Tetramethylolacetylendiharnstoff

Dimethylolglyoxalmonourein
Hexamethylentetramin
Glyoxal
Glutardialdehyd
N-Methylol-chloracetamid
1-(Hydroxymethyl)-5,5-dimethyl-hydantoin
1,3-Bis-(hydroxymethyl)-5,5-dimethylhydantoin
Imidazolidinylharnstoff
1-(3-Chlorallyl)-3,5,7-triaza-1-azonia-adamantan-chlorid
1,3-Bis-(β-ethylhexyl)-5-methyl-5-amino-hexahydropyrimidin
1,3,5-Tris-(hydroxyethyl)-1,3,5-hexahydrotriazin
1,2-Dibrom-2,4-dicyanobutan
5-Brom-5-nitro-1,3-dioxan
2-Brom-2-nitropropandiol
1,1'-Hexamethylen-bis-[5-(4-chlorphenyl)-biguanid]
4,4-Diaminodiphenoxypropan
2-Brom-2-nitro-propan-1,3-diol
Sorbinsäure und ihre Salze
p-Hydroxybenzoesäure und ihre Ester und Salze
Zink-2-pyridinethiol-N-oxid
2-[(Hydroxylmethyl)amino]-ethanol
Dithio-2,2'-bis(benzmethyl-amid)
5-Chlor-2-(2,4-dichlorphenoxy)-phenol
Thio-bis-(4-chlorphenol)
o-Phenyl-phenol
Chlormethyl-dijodmethylsulfon
p-Chlorphenyl-3-jodpropargyl-formal

Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I genutzt. Die so erhaltenen Verbindungen sind in den anschließenden Tabellen mit physikalischen Daten aufgeführt.

1. N-hydroxy-N-(2-propylphenyl)-2-chlornicotinsäureamid

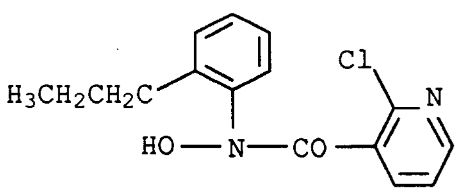

Zu einer Lösung von 15,1 g 2-n-Propylphenylhydroxylamin in 75 ml Ether-Petrolether-Gemisch (2:1) gibt man bei 0 °C 14 ml Wasser und 19,6 g Natriumhydrogencarbonat und tropft anschließend unter intensivem Vermischen 13,6 g 2-Chlornicotinsäurechlorid hinzu. Nach Rühren des Ansatzes bei Raumtemperatur über Nacht wird abgesaugt. Der Rückstand wird 15 Minuten in 10 % Natriumhydrogencarbonatlösung gerührt abgesaugt, in Essigester gelöst, getrocknet und das Lösungsmittel im Vakuum verdampft. Aus dem Rohprodukt (14,6 g) isoliert man nach Umkristallisieren aus Ethanol 12,5 g 2-Chlornicotinsäure-N-hydroxy-2-n-propylanilid vom Fp: 134-135 °C.

Tabelle 1

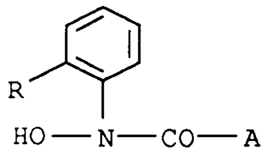

I

| Beispiel Nr. | R | A | physik. Daten |
|---|---|---|---|
| 1 | $CH(CH_3)_2$ | 2-Cl-pyridin-3-yl | 107-111°C |
| 2 | $CH_2CH_2CH_3$ | 2-Cl-pyridin-3-yl | 134-135°C |
| 3 | $CH_2CH(CH_3)_2$ | 2-Cl-pyridin-3-yl | Öl |
| 4 | Phenyl | 2-Cl-pyridin-3-yl | 112-115°C |
| 5 | $CH_2CH(CH_3)_2$ | 2-$CH_3$, 4-$CF_3$-thiazol-4-yl | Öl |
| 6 | Phenyl | 2-$CH_3$, 4-$CF_3$-thiazol-4-yl | 173-175°C |
| 7 | $CH_2CH(CH_3)_2$ | 2,4-$(CH_3)_2$-thiazol-4-yl | Öl |
| 8 | Phenyl | 2,4-$(CH_3)_2$-thiazol-4-yl | 58-62°C |

Beispiele zur biologischen Wirkung:

Wirksamkeit gegen Botrytis cinerea

Scheiben von grünen Paprikaschoten wurden mit einer wäßrigen Suspension [80 % Wirkstoff / 20 % Emulgator in der Trockenmasse] des Wirkstoffs tropfnaß gespritzt. Nach dem Abtrocknen des Spritzbelags wurden die Scheiben mit einer Sporensuspension [$1{,}7 \cdot 10^6$ Sporen pro ml; 2 % Biomalz; Wasser] des Pilzes Botrytis cinerea besprüht und anschließend 4 Tage bei 18 °C und hoher Luftfeuchtigkeit aufbewahrt.

Nach dieser Zeit wiesen die nicht mit Wirkstoff vorbehandelten Kontrollen einen Pilzbefall von 90 % auf, während die mit jeweils 500 ppm der Verbindungen Nr. 1 und 2 behandelten Paprika-Scheiben maximal zu 5 % befallen waren.

Bei einer Aufwandmenge von 1000 ppm der Verbindungen Nr. 1 und 2 wiesen die behandelten Paprika-Scheiben keinen Befall mehr auf, während Paprika-Scheiben, die mit 1000 ppm 2-Chlornicotinsäure-2-chloranilid behandelt waren, ebenso wie die unbehandelten Konterollen einen Befall von 90 % aufwiesen.

## Patentansprüche

**1.** N-Hydroxy-N-phenylcarbonsäureamide der Formel I

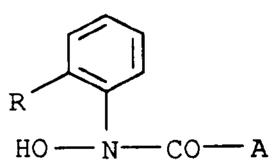

I

in der die Substituenten die folgende Bedeutung haben:

R $C_2$-$C_{12}$-Alkyl, $C_2$-$C_{12}$-Alkoxy, $C_3$-$C_{12}$-Alkenyl, $C_3$-$C_{12}$-Alkenyloxy, $C_3$-$C_6$-Alkinyl, $C_3$-$C_6$-Alkinyloxy, wobei diese Gruppen partiell oder vollständig halogeniert sein können;

$C_3$-$C_7$-Cycloalkyl, $C_4$-$C_7$-Cycloalkenyl, $C_3$-$C_7$-Cycloalkyloxy oder $C_4$-$C_7$-Cycloalkenyloxy, wobei diese Ringe ein bis drei $C_1$-$C_4$-Alkylgruppen tragen können;

Phenyl, welches ein bis fünf Halogenatome und/oder ein bis drei der folgenden Reste tragen kann: $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;

A ein cyclischer Rest aus der Gruppe der Formeln A1 bis A7

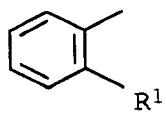
A1

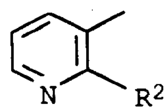
A2

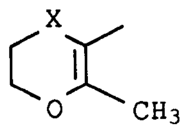
A3

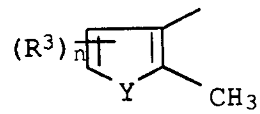
A4

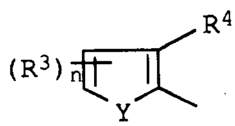
A5

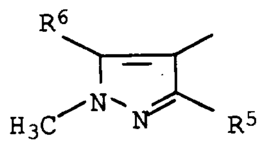
A6

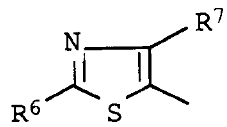
A7

in denen die Substituenten die folgende Bedeutung haben:

X -$CH_2$-, -S-, -SO- oder -$SO_2$-;

Y -O- oder -S-;

$R^1$, $R^2$, $R^4$, $R^5$ und $R^7$ Halogen, $C_1$-$C_4$-Alkyl oder $C_1$-$C_4$-Halogenalkyl;

$R^3$ und $R^6$ Wasserstoff, Halogen oder $C_1$-$C_4$-Alkyl;

n 1 oder 2, wobei die Reste $R^3$ verschieden sein können, wenn der Wert von n 2 beträgt.

**2.** N-Hydroxy-N-phenylcarbonsäureamide der Formel I, gemäß Anspruch 1, in der R die in Anspruch 1 gegebene Bedeutung hat und A für einen cyclischen Rest aus der Gruppe der Formeln A1 bis A7 steht, in denen X und Y die in Anspruch 1 gegebene Bedeutung und die Substituenten die folgende Bedeutung haben:

$R^1$ Halogen, Methyl oder $C_1$-Halogenalkyl;
$R^2$ Halogen oder $C_1$-Halogenalkyl;
$R^3$ Wasserstoff oder Methyl;
n 1 oder 2, wobei die Reste $R^3$ verschieden sein können, wenn der Wert von n 2 beträgt;
$R^4$ Halogen, oder Methyl;
$R^5$ Methyl oder $C_1$-Halogenalkyl;
$R^6$ Wasserstoff, Halogen oder Methyl;
$R^7$ Halogen, Methyl oder $C_1$-Halogenalkyl.

**3.** N-Hydroxy-N-phenylcarbonsäureamide der Formel I, gemäß Anspruch 1, in der R die in Anspruch 1 gegebene Bedeutung hat und A für einen cyclischen Rest aus der Gruppe der Formeln A1 bis A7 steht, in denen X und Y die in Anspruch 1 gegebene Bedeutung und die Substituenten die folgende Bedeutung haben:

$R^1$ Chlor, Brom, Jod, Methyl oder Trifluormethyl;
$R^2$ Chlor oder Trifluormethyl;
$R^3$ Wasserstoff oder Methyl;
n 1 oder 2, wobei die Reste $R^3$ verschieden sein können, wenn der Wert von n 2 beträgt;
$R^4$ Chlor oder Methyl;
$R^5$ Methyl, Difluormethyl oder Trifluormethyl;
$R^6$ Wasserstoff, Chlor oder Methyl;
$R^7$ Chlor, Methyl oder Trifluormethyl.

4. Verfahren zur Herstellung der Verbindungen I gemäß Anspruch 1, dadurch gekennzeichnet, daß man ein Carbonsäurehalogenid der Formel II

Hal-CO-A II

in der Hal für ein Halogenatom steht, in an sich bekannter Weise in Gegenwart einer Base mit einem N-Hydroxyanilin der Formel III

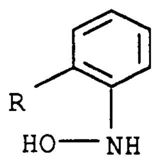

III

umsetzt.

5. Mittel zur Bekämpfung von Schadpilzen, enthaltend eine fungizide Menge einer Verbindung der Formel I gemäß Anspruch 1, 2 oder 3 und inerte Zusatzstoffe.

6. Verfahren zur Bekämpfung von Schadpilzen, dadurch gekennzeichnet, daß man die Schadpilze, ihren Lebensraum und/oder die von Schadpilzen freizuhaltenden Pflanzen oder Materialien mit einer fungizid wirksamen Menge einer Verbindung der Formel I gemäß Anspruch 1, 2 oder 3 behandelt.

7. Verwendung der Verbindungen I gemäß Anspruch 1, 2 oder 3 zur Bekämpfung von Schadpilzen.

8. Verwendung der Verbindungen I gemäß Anspruch 1, 2 oder 3 zur Bekämpfung von Botrytis.

Europäisches Patentamt

# EUROPÄISCHER RECHERCHENBERICHT

Nummer der Anmeldung

EP 93 11 4541
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| X | US-A-3 541 106 (VELSICOL CHEMICAL CO.) 17. November 1970<br>* Ansprüche 1-3 *<br>--- | 1-8 | C07D213/82<br>C07D277/56<br>A01N43/40<br>A01N43/78 |
| X | US-A-3 646 042 (VELSICOL CHEMICAL CO.) 29. Februar 1972<br>* Spalte 1, Zeile 62 - Spalte 1, Zeile 64 *<br>--- | 1-4 | |
| D,A | DE-A-2 417 216 (BASF AG) 6. November 1975<br>* gesamtes Dokument *<br>--- | 1-8 | |
| A | DE-A-1 642 224 (BASF AG) 13. April 1972<br>* gesamtes Dokument *<br>--- | 1-8 | |
| A | DE-C-1 642 232 (BASF AG) 11. August 1983<br>* gesamtes Dokument *<br>--- | 1-8 | RECHERCHIERTE SACHGEBIETE (Int. Cl.5 ) |
| A | J. CHEM. ENG. DATA<br>Bd. 22, Nr. 1, 1977,<br>Seiten 70 - 71<br>Y. K. AGRAWAL 'Synthesis of N-Arylhydroxamic Acids'<br>* Tabelle I *<br>--- | 1-8 | C07D<br>A01N |
| A | J. INDIAN CHEM. SOC.<br>Bd. 48, Nr. 8, 1971,<br>Seiten 753 - 756<br>V.K. GUPTA, S.G. TANDON 'Studies on Hydroxamic Acid. Preparation and Properties of N-Aryl Hydroxamic Acids Derived from Aromatic Carboxylic Acids'<br>* Tabelle I *<br>---<br>-/-- | 1-8 | |

Der vorliegende Recherchenbericht wurde für alle Patentansprüche erstellt

| Recherchenort | Abschlußdatum der Recherche | Prüfer |
|---|---|---|
| MUENCHEN | 03 FEBRUAR 1994 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 93 11 4541
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | ANALYST<br>Bd. 101, Nr. 1242, 1979,<br>Seiten 873 - 875<br>Y.K. AGRAWAL 'Non-aqueous Titration of Substituted N-o-Tolylbenzohydroxamic Acids'<br>* Tabelle I *<br>----- | 1-8 | |
| | | | RECHERCHIERTE SACHGEBIETE (Int. Cl.5 ) |

Der vorliegende Recherchenbericht wurde für alle Patentansprüche erstellt

| Recherchenort | Abschlußdatum der Recherche | Prüfer |
|---|---|---|
| MUENCHEN | 03 FEBRUAR 1994 | HERZ C.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)